(19) Europäisches Patentamt
European Patent Office
Office européen des brevets

(11) **EP 2 110 075 B1**

(12) **EUROPEAN PATENT SPECIFICATION**

(45) Date of publication and mention
of the grant of the patent:
**28.01.2015 Bulletin 2015/05**

(51) Int Cl.:
**A61B 5/0484** (2006.01)    **A61B 5/0496** (2006.01)

(21) Application number: **09155889.0**

(22) Date of filing: **23.03.2009**

(54) **A process and system for recording erg, perg and vep multifocal electrofunctional responses in real time**

Verfahren und System zur Echtzeit-Aufzeichnung von multifokalen ERG-, PERG- und VEP-Elektrofunktionalantworten

Procédé et système pour enregistrer des réponses électrofonctionnelles multifocales erg, perg et vep en temps réel

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **18.04.2008  IT FI20080081**

(43) Date of publication of application:
**21.10.2009  Bulletin 2009/43**

(73) Proprietor: **Costruzioni Strumenti Oftalmici C.S.O. S.r.l.**
**50010 Scandicci (Firenze) (IT)**

(72) Inventors:
• **Baglini, Claudio**
**56010, Migliarino Pisano (Pisa) (IT)**
• **Vestri, Gabriele**
**50142, Firenze (IT)**
• **Versaci, Francesco**
**59100, Prato (IT)**

(74) Representative: **Soldatini, Andrea et al**
**Società Italiana Brevetti S.p.A.**
**Corso dei Tintori, 25**
**50122 Firenze (IT)**

(56) References cited:
**WO-A-2006/106548      US-A- 4 846 567**
**US-A- 6 022 107       US-A1- 2007 115 869**

• **BEARSE M.A. AND SUTTER E.E.: "Imaging localized retinal dysfunction with the multifocal electroretinogram" J. OPT. SOC. AM., vol. 13, no. 3, March 1996 (1996-03), pages 634-640, XP007909157**
• **SUTTER E E: "The fast m-transform: a fast computation of cross-correlations with binary m-sequences" SIAM JOURNAL ON COMPUTING, SOCIETY FOR INDUSTRIAL AND APPLIED MATHEMATICS, US, vol. 20, no. 4, 1 August 1991 (1991-08-01), pages 686-694, XP000910312 ISSN: 0097-5397**
• **E.E. SUTTER ET AL.: "The Field Topography of ERG Components in Man", VISION RESEARCH, vol. 32, no. 3, 1992, pages 433-446,**

**Description**

The present invention refers to the field of ophthalmologic diagnosis, in particular that of major ocular pathologies like glaucoma, retinal anomalies and of sight, retinal degeneration of the retinal structure and retinal macular degeneration, and disorders affecting the optical nerve and the visual cortex. More specifically, the invention concerns a new process and system for recording ERG, PERG, VEP and multifocal electrofunctional responses.

Such a known diagnostic technique is based upon recording bioelectric responses generated by the retina, by the optical nerve and all of the cellular and nervous processes including the central visual cortex, as a consequence of a visual stimulus perceived by the patient. The aforementioned bioelectric responses are recorded, through suitable electrodes arranged at the level of the conjunctival fornix, cornea, or near to the central visual cortex by means of surface electrodes (of both eyes in the case of binocular recording), from which a biopotential can be obtained. This represents a measurement of the integrity of the visual system (density of cones, rods and cells connected to them, gangliocytes, retinal cells, nerve fibres and visual cortex) or of possible alterations or destructive actions already caused by the various pathologies.

With the current techniques (US patent n. 4,846,567 7/1989, Sutter et. al. - E. B. Brown et al. Contrast luminance as parameter defining.... Etc. Ophthalmic and physiological optic, Vol. 16 n. 1 Jan. 1996 pp.42-48 - E.E. SUTTER ET AL.: "The Field Topography of ERG Components in Man" VISION RESEARCH, Bd. 32, Nr. 3, 1992, Seiten 433-446) it is possible to record a number n of biopotentials (responses generated by different locations, not only retinal) suitable for allowing the topography of the retina or of the central visual cortex.

Although widely used in current ophthalmologic diagnosis, this technique has some limitations, the first of which is the lack of control of the retinal and/or consequently cortical areas really stimulated, and the instant feedback of the result of the bioelectric responses recorded. In order to stimulate and consequently record the biopotential generated by the different areas, techniques have been developed including the one documented in E.E. SUTTER ET AL.: "The Field Topography of ERG Components in Man" VISION RESEARCH, Bd. 32, Nr. 3, 1992, Seiten 433-446, based upon stimulation through "m-sequences" and the subsequent decoding by means of cross-correlation between stimulated area and consequent biopotential (retinal or cortical reaction) recorded. However, it requires very long execution times and allows possible artifacts (biopotentials induced by loss of focus or movements of the patient's eyes) to be monitored but does not allow them to be quickly corrected. This can contaminate the subsequent result, as widely documented in Guideline for basic Multifocal Electroretinography (mfERG) Documenta Ophthalmologica n. 106 pp.105-115, 2003.

Further improvements of the current technique are disclosed in US patent n. 6,022,107 8/2000 Ernst Kutschbach et. al., with which improvements are introduced such as the fragmentation of the m-sequence into steps lasting a shorter time that allow an intermediate evaluation point of the quality of the result, also allowing possible samples - judged to be unreliable thanks to a discarding automatism of the bioelectric responses exceeding predetermined size limits - to be repeated. Such a technique does, however, require waiting until the end of each cycle of each m-sequence for some repetitions, imposing long and pointless waiting times on the patient.

In M.A. BEARSE ET AL.: "Imaging localized retinal dysfunction with the multifocal electroretinogram" J. OPT. SOC. AM, Vol. 13, Nr. 3, March 1996, fast computation techniques are disclosed for shortening the duration of the analysis, but as long as the end of each cycle of a m-sequence is to be waited for, no satisfactory results can be expected.

The object of the present invention is to overcome the situation described above, by providing a system and an apparatus of the type referred to, capable of allowing further improved quality control of the result, eliminating the uncertainty of measurement due to the intrinsic variability of the result itself in conventional clinical practice.

This purpose is accomplished with the process for recording ERG, PERG, VEP and multifocal electrofunctional responses in real time according to the present invention, the essential characteristics of which are defined in the first of the attached claims. A system specifically adapted to carry out the process according to the invention has the essential characteristics defined in claim 4.

The characteristics and advantages of the process and system for recording ERG, PERG, VEP and multifocal electrofunctional responses in real time according to the present invention will become apparent from the following description of embodiments thereof, given as an example and not for limiting purposes, with reference to the attached drawings, in which:

- figures 1a, 1b show an arrangement, on the face and on the head of a patient, of electrodes intended to record the retinal and cortical biopotential according to the invention;
- figure 2 shows an outline representation of the hardware of a system according to the invention;
- figure 3 is a schematic representation of a different hardware system according to the invention;
- figures 4a to 4h show respective examples of pattern stimuli presented in the process according to the invention;
- figure 5 is an image representing a display that can be obtained by the medical operator with the apparatus according to figure 3.

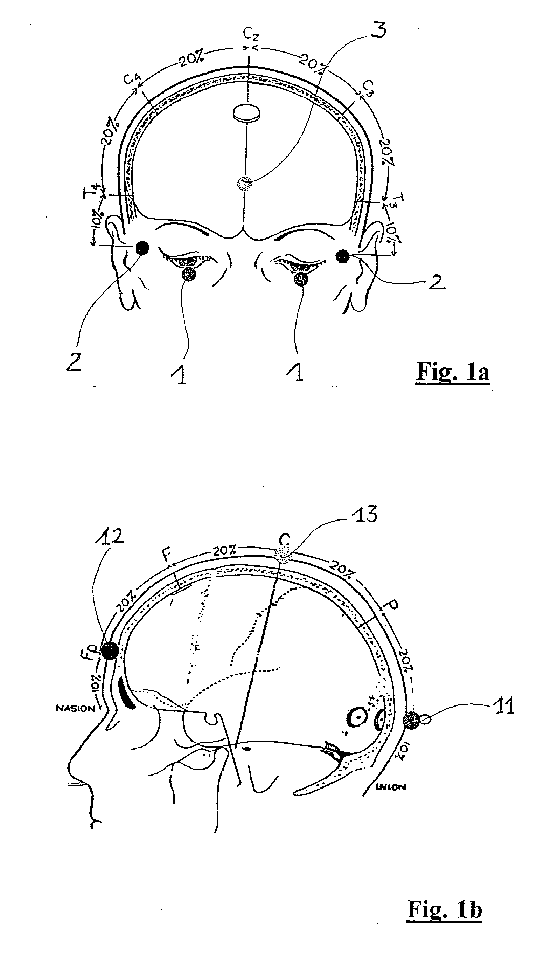

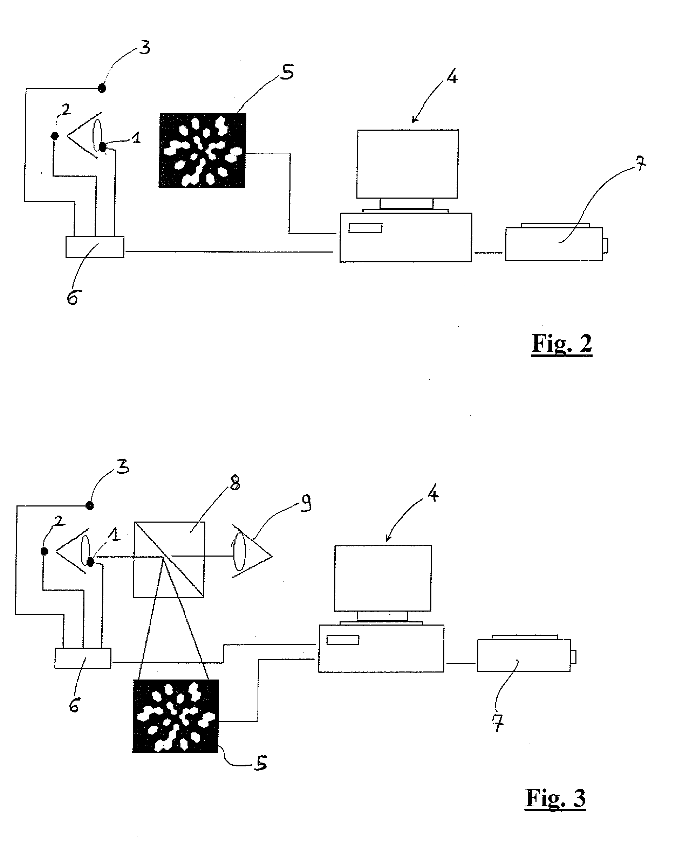

With reference to figures 1a, 1b, 2 and from 4a to 4h, according to the invention a configuration of electrodes is arranged on the face of a patient to be examined as in conventional ERG, PERG and VEP recording techniques. In particular, in case of retinal biopotential (figure 1 a), for each eye an exploring active electrode is inserted inside the lower conjunctival fornix (positions 1) or on the corneal surface of the eye itself, coming into contact with the eyeball or also at the level of the central visual cortex by means of surface electrodes, so as to pick up a bioelectric signal generated by the stimulated areas. Preferably, in order to record the retinal biopotential, the electrode is of the so-called HK LOOP type, comprising a very thin silver wire coated with a Teflon sheath on which some incisions are made to allow contact with the eyeball.

This type of electrode does not interfere with the patient's vision, also allowing him to comfortably blink, these being characteristics that are not found, for example, in the use of common corneal electrodes. Moreover, a better contact impedance between the electrode and the patient, and therefore a more reliable contact is obtained than cutaneous electrodes, possible refractive problems connected to the use of corneal electrodes is avoided, and it is possible to record the bioelectric signal with a very high signal/noise ratio, for which reason the time taken for an examination is substantially reduced (the number of samples to be taken is reduced).

A reference electrode is positioned, following suitable cleaning of the skin, at the level of the outer temporal corner of each eye (position 2, figure 1 a), and comprises a silver or silver chloride disc to which a special electroconductive paste is added so as to assist the contact with the patient's skin. A common electrode, of the same type as the reference electrode, is finally arranged at the center of the patient's forehead (position 3). In case of cortical detection, again as an example, the positioning of the exploring active electrode, reference electrode, and common electrode, follows the configuration respectively indicated by the positions 11, 12 and 13 of figure 1 b.

The patient, with the aforementioned electrodes already applied, is positioned a distance of about 30 cm from an apparatus for performing the examination, with the chin resting on an adjustable chin-rest. The apparatus, shown in figure 2, comprises a personal computer 4 with a software system suitable for simultaneously managing a generation of a sequence of visual stimuli shown in a screen 5 arranged at the height of the patient's eyes, and for carrying out a consequent recording of the biopotential. In accordance with what has already been disclosed in conventional apparatuses, the average brightness of the screen is calibrated according to the type of examination according to preselected values.

The recording of the biopotential takes place, in the illustrated embodiment, through the use of a differential amplifier 6 connected both to the personal computer 4 and to all the electrodes 1, 2, 3 (or else 11, 12, 13). Such a differential amplifier is characterized by a passband of between 0.1 Hz and 3000 Hz and an amplification factor of between 50,000 and 500,000 V/v. A 16 bit A/D converter (not shown) digitizes the signal, all according to the known conventional applications.

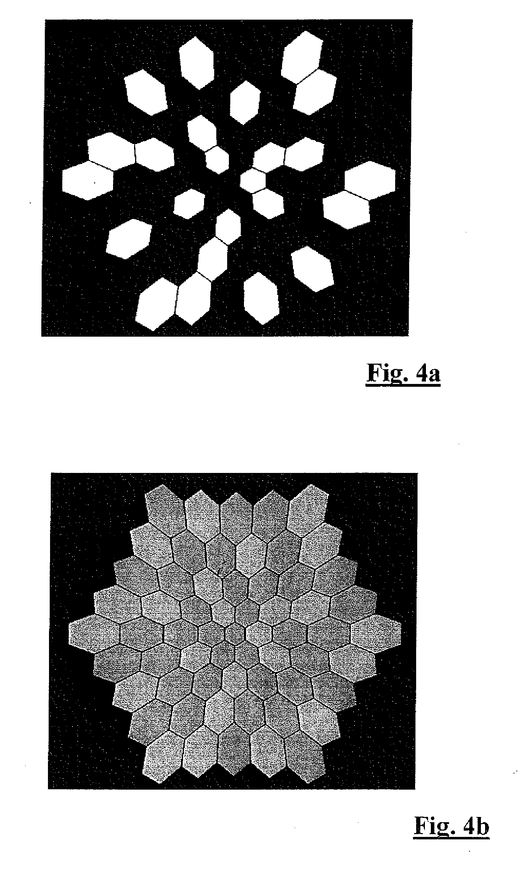

The software residing in the personal computer, above and beyond allowing the recorded biopotential to be processed, this processing being printable with a printer 7, as stated controls the generation of pattern stimuli presented on the screen 5. Said stimuli are configured on the screen as shown, for example, in figures 4a to 4d. The patient is shown a sequence of cell patterns, i.e. a group of visual stimuli having various spatial and temporal forms. In synchrony with the stimulus, the system acquires the sampled signal to be processed (bioelectric response associated with the presentation of a visual stimulus) from the electrodes suitably positioned on the patient. Each cell on the screen is modulated based upon a corresponding binary m-sequence, in the way explained hereafter.

The ultimate goal of the examination is to associate a bioelectric response with every cell belonging to the stimulation pattern, i.e. to the retinal portion or cortical projection associated with such a cell.

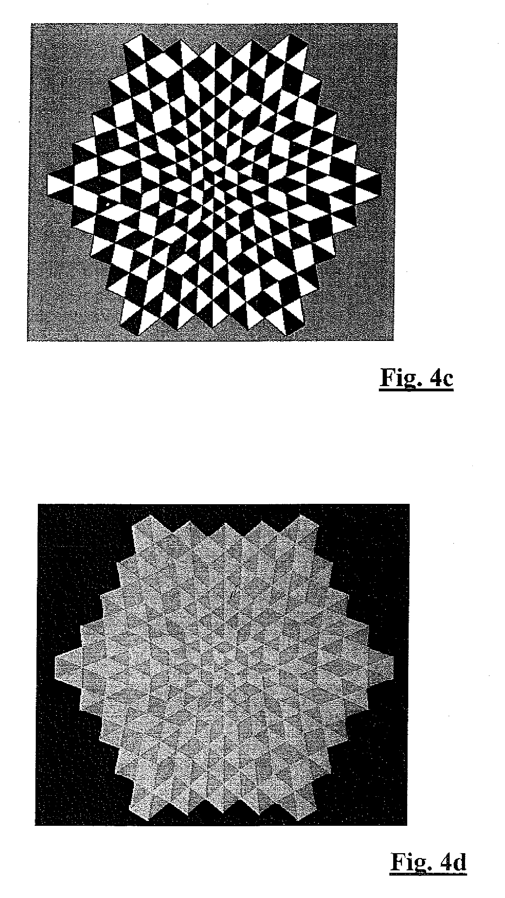

For MF-ERG examination, the term "activated" must be interpreted as "lit" and "deactivated" must be interpreted as "not lit" (figure 4a, in which some cells are lit, others not). For MF PERG and VEP examinations, on the other hand, the word "activated" must be interpreted as placed in the "normal pattern condition", and "deactivated" must be interpreted as placed in the "inverse pattern condition" (figure 4c, in which some cells have a normal pattern, other inverse pattern).

As represented by figures 4b and 4d, it is also possible to modify the characteristics of the images, for example color, brightness, contrast but also shape, spatial size, temporal alternation, distortion, visual angle subtended. In this way, the n retinal areas or relative cortical areas that result from the characteristic of the type of multifocal stimulus used for the stimulation of the patient can be stimulated independently.

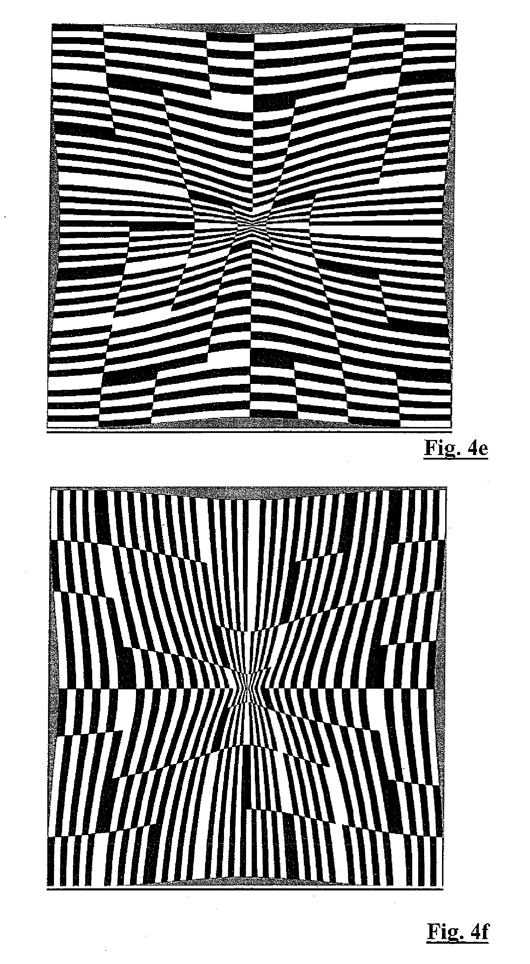

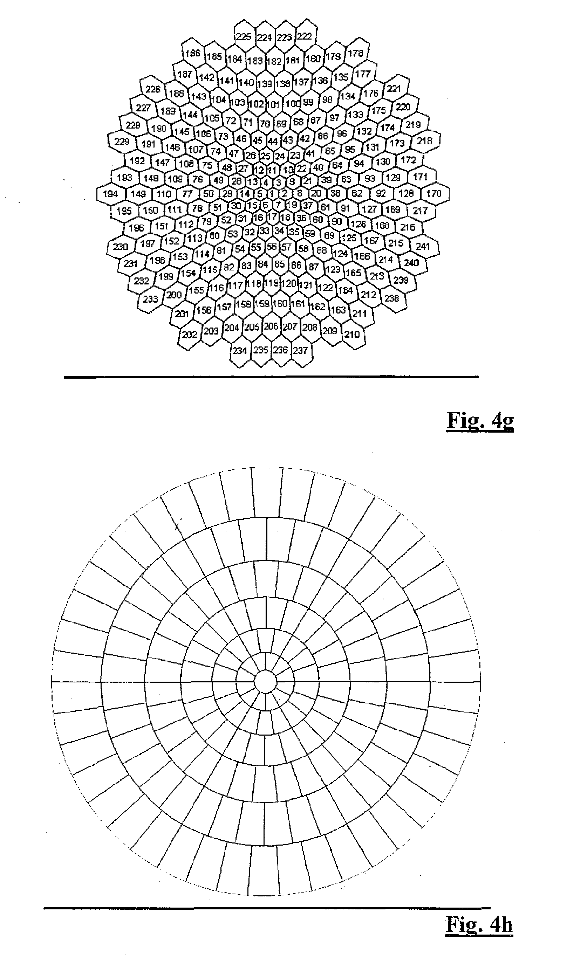

Furthermore, figures 4e and 4f show a different set of stimuli suitable for selectively stimulating visual areas or retinal and cortical receptive fields, both horizontal and vertical. Figure 4g represents a different stimulus set made up of a plurality of cells (hexagons) suitable for stimulating a different amount of visual areas or retinal and cortical receptive fields. Finally, figure 4h shows a different set of images made up of a plurality of cells divided into sectors of concentric rings further divided into white-black elements or contrasting colors suitable for stimulating visual areas or retinal and cortical receptive fields

Returning specifically to the control of the stimulation, the recording of the biopotential and, therefore, the measurement of the retinal or cortical biopotential, specifically for the n areas stimulated, takes place as follows.

The aforementioned m-sequence is a sequence of symbols 1 and -1 of length $N = 2^s - 1$ , s being a positive whole number. If the m-sequence is N symbols long it is possible to manage a pattern of M cells (M <= N). Each cell is associated with an m-sequence N symbols long that can be obtained by cyclically translating a mother m-sequence. The various m-sequences generated by cyclic translation by a mother m-sequence are pseudo-orthogonal, according to the example diagram shown below (s = 3, N = 7).

| Seq. 1 | 0 | 1 | 0 | 0 | 1 | 1 | 1 |
| Seq. 2 | 1 | 0 | 1 | 0 | 0 | 1 | 1 |
| Seq. 3 | 1 | 1 | 0 | 1 | 0 | 0 | 1 |
| Seq. 4 | 1 | 1 | 1 | 0 | 1 | 0 | 0 |
| Seq. 5 | 0 | 1 | 1 | 1 | 0 | 1 | 0 |
| Seq. 6 | 0 | 0 | 1 | 1 | 1 | 0 | 1 |
| Seq. 7 | 1 | 0 | 0 | 1 | 1 | 1 | 0 |

In the following time-scale:

$Ts$ is the period of a symbol time of the m-sequence, i.e. of a 1 or -1. T is the period of an entire m-sequence, i.e. the period of a symbol time multiplied by the length of the m-sequence N. Be it assumed that k periods $Ts$ have passed from the start of the m-sequence, if the q-th m-sequence associated with the q-th cell contains a 1 in the k-th position, the cell of the pattern on the screen shall be activated, otherwise it will be deactivated.

The time period between two stimuli is selected so as to be great enough to take up the entire bioelectric response in such a time.

At the end of each m-sequence, i.e. after each period of length $N*Ts$, it is possible to calculate the response of every cell based upon the data gathered during the execution time of the m-sequence just ended. Assuming that one is interested in the response of the q-th cell, with which the q-th m-sequence obtainable through a cyclic translation of q symbols of the mother m-sequence is associated. The data of the overall response of each part highlighted in the time-scale shown above can be cross-correlated with the m-sequence associated with the q-th cell, to obtain the current response of the q-th cell due to the m-sequence just ended.

If a time period equal to a whole number n of m-sequences has passed, it is possible to calculate the average response of each cell using the responses of each cell derived from the data collected during the first, second, up to the n-th stimulation m-sequence.

According to a specific and characteristic aspect of the present invention, contrary to what was done previously, the calculation of the response of every cell is updated at the end of every symbol time equal to T/N. In this way, it is possible to follow the evolution of the calculated response of each cell in real time without having to wait for the end of an m-sequence. Consequently, there is a substantial benefit in terms of saving time and also immediately displaying the effectiveness of the result and possible errors.

The above can be explained in detail in the following example. Be it assumed that k is equal to a whole number of time periods Ts that follow the end of the previous m-sequence after which it is wished to obtain an update of the wave-forms of the responses of the various cells. Be it then considered the part of overall response of length N*Ts that goes from the moment $T_{refresh}$ at which an update is wanted, up to $T_{refresh}$ - N*Ts.

Supposing that one is interested in the response of the q-th cell with which at the beginning the q-th m-sequence obtainable through a cyclic translation of q symbols of the mother m-sequence is associated, the data extracted from the overall response (from $T_{refresh}$ up to $T_{refresh}$ - N*Ts = $T_{refresh}$ - T) must be cross-correlated not with the m-sequence associated with the q-th cell but with a version thereof cyclically delayed by k symbols to obtain the current response of said q-th cell due to the N most recent stimuli (generated in a time period T). See the example in the following diagram in which for each cell the sequences to be cross-correlated with the overall response acquired in the time period from $T_{refresh}$ up to $T_{refresh}$ - N*Ts are represented. In this example, k is equal to 4 and the set of m-sequences used is the one previously represented.

This partial result can be used to calculate the average response associated with the q-th cell in combination with the partial results calculated previously for this same cell.

The calculated wave-forms associated with the various cells can be directly interpreted or else associated with a pattern made up of luminous, isoluminant or gray-scale chromatic stimuli that mirrors the matrix of the stimuli.

To sum up, the displaying in real time of the results allows the operator or automatically allows the processor to:

- monitor possible loss of attention by the patient or possible artifacts that can contaminate and jeopardize the outcome of the examination;
- interrupt the acquisition cycle of the biopotential at any moment;
- repeat only the stimulus part where an artifact of the biopotential associated with the responses generated by specific

EP 2 110 075 B1

cells has been identified.

The time taken for the examination is thus significantly reduced with the result of optimizing the attention, learning and fatigue capability of the patient, having the advantage, as instantaneous evaluation element, of an intrinsic coefficient of variation (CV) and a standard error of the mean (SEM) of the result obtained in real time, and at the same time the qualitative analysis of the result.

Regarding this last point, it is clear that, according to the selected analysis strategy, during the acquisition process the bioelectric responses currently calculated

$$\overline{A} = \sum_{0}^{N_c-1} {}_i A_i \qquad \sigma_A = \sqrt{\frac{1}{N_c-1}\sum_{0}^{N_c-1}(A_i - \overline{A})^2}$$

could differ from the corresponding previous ones. The coefficient of variation (CV) provides as instantaneous information, the standard deviation of the measurements, i.e. the root mean square $\sigma_A$ of the responses calculated in successive time periods purged of the mean value $\overline{A}$.

The standard error of the mean (SEM) equal to the standard deviation of the samples measured divided by the root of the number Nc of samples measured, on the other hand, is used to evaluate the reliability of the measurement.

$$CV = \frac{\sigma_A}{\overline{A}} \qquad SEM = \frac{\sigma_A}{\sqrt{N_c}}$$

It should be noted that such a parameter cannot be clinically tested but provides the clinic operator with data on how much the phenomenon has been correctly characterized in statistical terms.

Advantageously, it is possible to compare the result obtained from the patient examined with a normative database derived from extensive statistics collected on samples of normal subjects, capable of providing an indication of the deviation of the subject compared to the average of the normal subjects in relation to age. For example, a statistical test carried out on 154 healthy eyes was performed in order to define the normal ranges for the parameters of size and latency of the bioelectric responses correlated with the retinal surface stimulated by the various electrofunctional examinations. The statistical analysis was carried out on a population of normal subjects and in order to establish the existence and the correlation with the age of the patient. The examinations considered belong to a variegated population of patients from a minimum age of 12 years old up to a maximum of 87. The results obtained show the dependence of the size and latency upon the age of the subject examined. Such normative data, or other analogous data, after having been evaluated from the statistical point of view, can be inserted as a physiological parameter of "normality" into the apparatus, in order to make the user able to quickly and effectively consult it.

The present invention thus makes it possible to obtain a result immune from intrinsic factors of variability of the conventional technique like for example problems linked to the loss of focus or eye movements. The apparatus described above as an example allows the examination of a single eye or of both eyes of the patient simultaneously. According to a different embodiment, schematized by figure 3 (in which part identical or corresponding to those already described for the first embodiment are indicated with the same reference numerals), on the other hand, it is only necessary to examine one eye at a time. Moreover, such a solution ensures even greater precision of the results, avoiding the contamination of the biopotential with possible unreliable recordings.

In particular, the apparatus of figure 3 differs from the previous one in that a mirror or prism for optical beam separation, also known as beam splitter, of a *per se* known type and indicated at 8, is arranged between the eye of the patient and the pattern stimulator (CRT, CCD, LED, OLED, PLASMA). The beam splitter, arranged in a suitable position on the optical path, allows the patient to be shown a visual stimulus and at the same time allows the operator to be shown the image of the relevant portion of the patient's eye.

The medical operator, symbolized in the figures by the eye 9, directly or by means of a camera in turn connected to a monitor, can thus observe the retina base of the patient during the course of the examination thanks to an ophthalmoscope (not shown), ensuring that the stimulus is projected exactly at the centre of the fovea.

Therefore, he is able to instantly make corrections, interrupting and then continuing the stimulus associated with the response of the stimulated area (this thanks to the real time control according to the present invention), as a consequence of a loss of focus of the patient on the stimulus, for example due to an eye movement, blinking or attention difficulties in following the examination itself. The operator can also temporarily interrupt the recording in the case of the patient experiencing slight momentary difficulties as the examination progresses. The exact projection of the stimulus in the foveal area thus allows the occurrence of unreliable measurements caused by the patient having difficulties during the examination to be minimized.

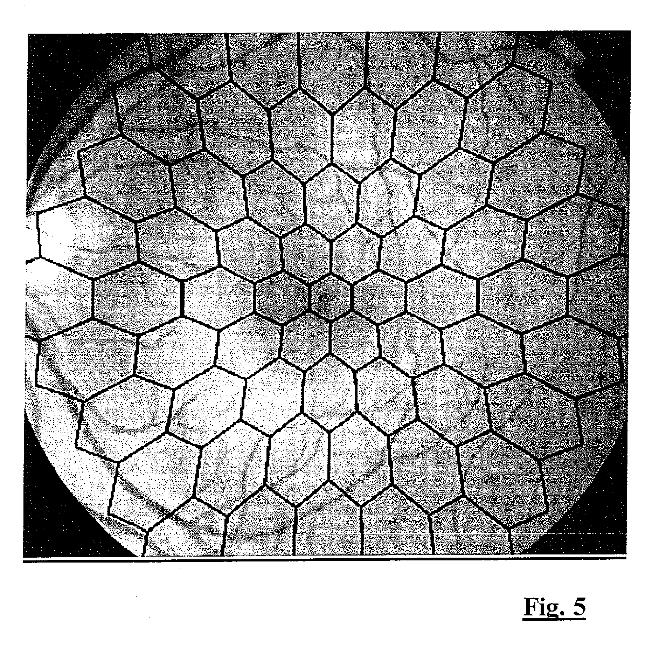

In a particularly advantageous embodiment, the beam splitter, the ophthalmoscope, the real time photography of the fundus oculi and the focusing of the stimulus on the desired retinal areas, the system for collimation, pointing and correcting the refractive error or ametropia of the patient, and a suitable optical system for projecting the pattern are stably integrated in a single optical-mechanical-electronic assembly, so that the view field of the ophthalmoscope and the projection field of the pattern exactly and unequivocally match up. Indeed, figure 5 provides an idea of the matching up of the response generated by each individual cell with the image of the fundus oculi of the patient examined.

With these solutions, therefore, also the conventional comparison between the response obtained from the patient with the normality data correlated with the age of the subject and obtainable from clinical testing, can be carried out much more precisely and reliably, which in any case allows the coefficient of variability connected to the current technique to be substantially reduced. The result of the diagnosis is really independent of the operator's ability and the patient's will, as well as of all other factors and sources of variability described above.

In both embodiments, the possibility of creating an archive of the results obtained for subsequent diagnosis makes it possible to monitor the progress of the pathology over time, and the possible effectiveness of a pharmacological treatment.

Variants and/or modifications can be brought to the process and system for recording ERG, PERG, VEP and multifocal electrofunctional responses in real time according to the present invention without for this reason departing from the scope of protection of the invention itself, as defined by the claims.

**Claims**

1. A process for determining the topography of the bioelectric response signals of a visual system of a patient, said system including retina, optical nerve or their projection at the level of the central cortex, comprising the steps of: - stimulating visually said patient through a surface arranged in front of the patient's eye, in which an image consisting of a plurality of cells is displayed as stimulation, each cell being activated or deactivated according to a corresponding digital time function represented by a cyclic succession of binary m-sequences of duration (T) formed from a plurality of activation symbols (N) each having a duration (Ts), the m-sequences of the various cells being obtained cyclically from a mother m-sequence; - determining the total bioelectric response of the visual system, the response associated with each cell being determined by the total response of the visual system by means of a cross-correlation with a suitable translated version of a mother m-sequence, **characterized in that** the calculation of the response of each cell is updated at the end of each symbol time (Ts), the updating being carried out, after a certain number (k) of symbol times (Ts) to obtain the response of a q-th cell associated with an m-sequence translated by a number (q) of symbols from the mother m-sequence, by cross-correlating the data extracted from the overall response not with said translated m-sequence, but with a version thereof cyclically delayed by said number (k) to obtain the current response of said q-th cell due to a number (N) of most recent stimuli, thereby allowing to follow the evolution of the calculated response of each cell in real time without awaiting the end of an m-sequence.

2. The process according to claim 1, wherein the calculated wave-forms associated with the various cells are directly interpreted or else associated with a pattern made up of luminous, isoluminant or gray-scale chromatic stimuli that mirrors the matrix of the stimuli.

3. The process according to any of the previous claims, wherein the result obtained by the patient examined is compared with a normative database comprising results obtained on normal subjects, in order to provide an indication of the deviation of the subject compared to the average of the normal subjects in relation to age.

4. A system for determining the topography of the bioelectric response signals of a visual system of a patient including retina, optical nerve or their projection at the level of the central cortex, following visual stimulation, comprising display means (5) adapted to be arranged in front of a patient's eye, processing means (4) connected to said display means (5) for displaying an image, as stimulation, consisting of a plurality of cells, each cell being activated or deactivated according to a corresponding digital time function represented by a cyclic succession of binary m-sequences of duration (T) formed from a plurality of activation symbols (N) each having a duration (Ts), the m-sequences of the various cells being obtained cyclically from a mother m-sequence, the system further comprising sensor and amplifier means (1, 2, 3, 6) for determining the total bioelectric response of the visual system, and for recording it on said processing means, thereby the response associated with each cell being determined by the total response of the visual system by means of a cross-correlation with a suitable translated version of a mother m-sequence, **characterized in that** said processing means (4) is configured to update the calculation of the response of each cell at the end of every symbol time (Ts), wherein said update is carried out after a certain number (k) of symbol times (Ts) to obtain the response of a q-th cell associated with an m-sequence translated by a number (q) of symbols from the mother m-sequence, by cross-correlating the data extracted from the overall response with a version of said translated m-sequence cyclically delayed by said number (k) to obtain the current response of said q-th cell due to a number (N) of most recent stimuli, allowing to follow the evolution of the calculated response of each cell in real time without having to wait for the end of an m-sequence.

5. The system according to claim 4, wherein said processing means (4) is configured to interpret the calculated waveforms associated with the various cells directly or else associated with a pattern made up of luminous, isoluminant or gray-scale chromatic stimuli that mirrors the matrix of the stimuli.

6. The system according to claim 4 or 5, wherein said processing means (4) comprise storage means of a normative database comprising results obtained on normal subjects, and wherein the processing means is configured to compare the result obtained from the patient examined with said database, in order to provide an indication of the deviation of the subject compared to the average of the normal subjects in relation to age.

7. The system according to any of the claims 4 to 6, wherein between said patient and said display means (5) optical beam separator means (8) are arranged, suitable for allowing said patient to observe said stimulation reflected by said divider means instead of directly from said display means, thereby allowing a medical operator, for control purposes, to observe the retina base of the patient during the course of the examination itself.

8. The system according to claim 7, further comprising ophthalmoscopic means to observe the retina base of the patient during the course of the examination, said optical beam separator means (8) and said display means (5) being stably integrated in a single optical-mechanical assembly with said ophtalmoscopic means.

9. A computer program for instructing a computer to perform the process according to any of the claims 1 to 3, when carried out in a system according to claim 4.

10. The computer program according to claim 9, **characterized in that** it is stored on a memory support.

11. The computer program according to claim 9, **characterized in that** it is housed on the processing means of the system according to any of the claims 5 to 8.

**Patentansprüche**

1. Verfahren zum Bestimmen der Topographie der bioelektrischen Antwortsignale eines visuellen Systems eines Patienten, welches System Retina, Optiknerven oder ihre Projektion auf die Ebene des zentralen Kortex enthält, enthaltend die Schritte: - visuelles Anregen des Patienten durch eine vor dem Auge des Patienten angeordnete Oberfläche, auf der ein Bild, das aus einer Mehrzahl von Zellen besteht, als Anregung angezeigt wird, wobei eine Zelle entsprechend einer digitalen Zeitfunktion aktiviert oder deaktiviert wird, die durch eine zyklische Abfolge von binären m-Sequenzen einer Dauer (T) repräsentiert wird, die aus einer Mehrzahl von Aktivierungssymbolen (N) gebildet wird, von denen jedes eine Dauer (Ts) hat, wobei die m-Sequenzen der verschiedenen Zellen zyklisch von einer Mutter-m-Sequenz erhalten werden; - Bestimmen der totalen bioelektrischen Antwort des visuellen Systems, wobei die Antwort, die zu jeder Zelle gehört, durch die totale Antwort des visuellen Systems mittels einer Kreuzkorrelation mit einer geeigneten übersetzten Version einer Mutter-m-Sequenz bestimmt wird, **dadurch gekennzeichnet, dass** die Berechnung der Antwort jeder Zelle am Ende jeder Symbolzeit (Ts) aktualisiert wird, wobei das Aktualisieren nach einer bestimmten Anzahl (k) von Symbolzeiten (Ts), um die Antwort einer q-ten Zelle zu erhalten, die zu einer m-Sequenz gehört, übersetzt durch eine Anzahl (q) von Symbolen von der Mutter-m-Sequenz, durch Kreuzkorrelation der Daten ausgeführt wird, die von der Gesamtantwort nicht innerhalb der m-Sequenz extrahiert wurden, aber mit einer Version davon, die zyklisch um die Anzahl (k) verzögert wurde, um die gegenwärtige Antwort der q-ten Zelle aufgrund einer Anzahl (N) von jüngsten Anregungen zu erhalten, wodurch es gestattet ist, der Evolution der berechneten Antwort jeder Zelle in Echtzeit zu folgen, ohne das Ende einer m-Sequenz abzuwarten.

2. Verfahren nach Anspruch 1, wobei die berechneten Wellenformen, die zu den verschiedenen Zellen gehören, direkt interpretiert werden mit oder anderweitig zugeordnet werden zu einem Muster, das aus leuchtenden, isoluminanten oder chromatischen Graukeil-Anregungen besteht, die die Matrix der Anregungen widerspiegeln.

3. Verfahren nach einem der vorhergehenden Ansprüche, wobei das Ergebnis, das durch den untersuchten Patienten erhalten wird, mit einer normativen Datenbank verglichen wird, die Ergebnisse enthält, die an normalen Subjekten erhalten wurden, um eine Indikation der Abweichung des Subjekts verglichen mit dem Durchschnitt der normalen Subjekte in Relation zum Alter bereit zu stellen.

4. System zum Bestimmen der Topographie der bioelektrischen Antwortsignale eines visuellen Systems eines Patienten, das Retina, Optiknerven oder ihre Projektion auf die Ebene des zentralen Kortex enthält, folgend auf eine visuelle Anregung, enthaltend Anzeigeeinrichtungen (5), die ausgelegt sind, um vor dem Auge des Patienten angeordnet zu werden, Verarbeitungseinrichtungen (4), die mit den Anzeigeeinrichtungen (5) verbunden sind, um ein Bild als eine Anregung anzuzeigen, das aus einer Mehrzahl von Zellen besteht, wobei jede Zelle entsprechend einer digitalen Zeitfunktion aktiviert oder deaktiviert wird, die durch eine zyklische Abfolge von binären m-Sequenzen einer Dauer (T) repräsentiert wird, die aus einer Mehrzahl von Aktivierungssymbolen (N) gebildet wird, von denen jedes eine Dauer (Ts) hat, wobei die m-Sequenzen der verschiedenen Zellen zyklisch von einer Mutter-m-Sequenz erhalten werden, wobei das System ferner Sensor- und Verstärkereinrichtungen (1, 2, 3, 6) zum Bestimmen der totalen bioelektrischen Antwort des visuellen Systems und zu seiner Aufzeichnung auf den Verarbeitungs-einrichtungen enthält, wodurch die Antwort, die zu jeder Zelle gehört durch die totale Antwort des visuellen Systems mittels einer Kreuzkorrelation mit einer geeigneten übersetzten Version einer Mutter-m-Sequenz bestimmt wird, **dadurch gekennzeichnet, dass** die Verarbeitungseinrichtungen (4) konfiguriert sind, um die Berechnung der Antwort jeder Zelle am Ende jeder Symbolzeit (Ts) zu aktualisieren, wobei das Aktualisieren nach einer bestimmten Anzahl (k) von Symbolzeiten (Ts), um die Antwort einer q-ten Zelle zu erhalten, die zu einer m-Sequenz gehört, übersetzt durch eine Anzahl (q) von Symbolen von der Mutter-m-Sequenz, durch Kreuzkorrelieren der Daten, die von der Gesamtantwort extrahiert wurden, mit einer Version der übersetzten m-Sequenz ausgeführt wird, die zyklisch um die Anzahl (k) verzögert wurde, um die gegenwärtige Antwort der q-ten Zelle aufgrund einer Anzahl (N) von jüngsten Anregungen zu erhalten, wodurch es gestattet ist, der Evolution der berechneten Antwort jeder Zelle in Echtzeit zu folgen, ohne das Ende einer m-Sequenz abwarten zu müssen.

5. Verfahren nach Anspruch 4, wobei die Verarbeitungseinrichtungen (4) konfiguriert sind, um die berechneten Wellenformen, die zu den verschiedenen Zellen gehören, direkt oder anderweitig zugeordnet zu interpretieren, mit einem Muster, das aus leuchtenden, isoluminanten oder chromatischen Graukeil-Anregungen besteht, die die Matrix der Anregungen widerspiegeln.

6. System nach Anspruch 4 oder 5, wobei die Verarbeitungseinrichtungen (4) Speichereinrichtungen einer normativen Datenbank enthalten, die Ergebnisse enthält, die an normalen Subjekten erhalten wurden, und wobei die Verarbeitungseinrichtungen konfiguriert sind, um das Ergebnis, das durch den untersuchten Patienten erhalten wurde, mit der Datenbank zu vergleichen, um eine Indikation der Abweichung des Subjekts verglichen mit dem Durchschnitt der normalen Subjekte in Relation zum Alter bereit zu stellen.

7. System nach einem der Ansprüche 4 bis 6, wobei zwischen dem Patienten und der Anzeigeeinrichtung (5) optische Strahlseparatoreinrichtungen (8) angeordnet sind, die geeignet sind, um es dem Patienten zu gestatten, die Anregung, die von den Teilereinrichtungen reflektiert werden, statt direkt von den Anzeigeeinrichtungen zu beobachten, wodurch es einem medizinischen Bediener zu Kontrollzwecken gestattet wird, die Retina-Basis des Patienten während des Verlaufs der Untersuchung selbst zu beobachten.

8. System nach Anspruch 7, ferner enthaltend ophtalmoskopische Einrichtungen, um die Retina-Basis des Patienten während des Verlaufs der Untersuchung zu beobachten, wobei die optischen Strahlseparatoreinrichtungen (8) und die Anzeigeeinrichtungen (5) fest in einen einzigen optischmechanischen Aufbau mit den ophtalmoskopischen Einrichtungen integriert sind.

9. Computerprogramm, um einen Computer zu instruieren, das Verfahren gemäß einem der Ansprüche 1 bis 3 durchzuführen, wenn es in einem System gemäß Anspruch 4 ausgeführt wird.

10. Computerprogramm nach Anspruch 9, **dadurch gekennzeichnet, dass** es auf einem Speicherhalter gespeichert ist.

11. Computerprogramm nach Anspruch 9, **dadurch gekennzeichnet, dass** es auf den Verarbeitungseinrichtungen des Systems nach einem der Ansprüche 5 bis 8 untergebracht ist.

**Revendications**

1. Un procédé pour déterminer la topographie des signaux de réponse bioélectrique d'un système visuel d'un patient, ledit système comprenant une rétine, un nerf optique ou leur projection au niveau du cortex central, comprenant les étapes suivantes : stimulation visuelle du dit patient via une surface disposée devant l'oeil du patient, dans laquelle est affichée comme stimulation une image consistant en une pluralité de cellules, chaque cellule étant activée ou désactivée selon une fonction temporelle digitale correspondante représentée par une succession cyclique des m-séquences binaires de durée (T) formées à partir d'une pluralité de symboles d'activation (N) chacun ayant une durée (Ts), les m-séquences des diverses cellules étant obtenues cycliquement à partir d'une m-séquence mère ; détermination de la réponse bioélectrique totale du système visuel, la réponse liée à chaque cellule étant déterminée par la réponse totale du système visuel au moyen d'une corrélation croisée avec une version translatée appropriée d'une m-séquence mère, **caractérisé en ce que** le calcul de la réponse de chaque cellule est actualisé à la fin de chaque temps de symbole (Ts), l'actualisation étant effectuée, après un certain nombre (k) de temps de symbole (Ts) pour obtenir la réponse d'une cellule q-th associée à une m-séquence translatée par un nombre (q) de symboles à partir de la m-séquence mère, en corrélant de façon croisée les données extraites de la réponse globale non pas avec ladite m-séquence translatée, mais avec une version de celle-ci cycliquement retardée par ledit nombre (k) pour obtenir la réponse actualisée de ladite cellule q-th due à un nombre (N) de stimulus les plus récents, permettant ainsi de suivre l'évolution de la réponse calculée de chaque cellule en temps réel sans attendre la fin d'une m-séquence.

2. Le procédé selon la revendication 1, où les formes d'ondes calculées associées aux diverses cellules sont directement interprétées ou associées à un motif de stimulus chromatiques lumineux, isoluminants ou d'échelles de gris qui reflète la matrice des stimulus.

3. Le procédé selon l'une quelconque des revendications précédentes, dans lequel le résultat obtenu par le patient examiné est comparé à une base de données normative comprenant des résultats obtenus sur des sujets normaux, afin de fournir une indication de la déviation du sujet par rapport à la moyenne des sujets normaux en fonction de l'âge.

4. Un système pour déterminer la topographie des signaux de réponse bioélectrique d'un système visuel d'un patient comprenant une rétine, un nerf optique ou leur projection au niveau du cortex central, à la suite d'une stimulation visuelle, comprenant des moyens d'affichage (5) adaptés pour être positionnés devant un oeil du patient, des moyens de traitement (4) reliés aux dits moyens d'affichage (5) pour afficher une image, comme stimulation, consistant en une pluralité de cellules, chaque cellule étant activée ou désactivée selon une fonction temporelle digitale correspondante représentée par une succession cyclique de m-séquences binaires de durée (T) formées à partir d'une pluralité de symboles d'activation (N) ayant chacun une durée (Ts), les m-séquences des diverses cellules étant obtenues cycliquement à partir d'une m-séquence mère, le système comprenant en outre des moyens capteurs et amplificateurs (1, 2, 3, 6) pour déterminer la réponse bioélectrique totale du système visuel, et pour l'enregistrer dans lesdits moyens de traitement, selon quoi la réponse associée à chaque cellule est déterminée par la réponse totale du système visuel au moyen d'une corrélation croisée avec une version translatée adaptée d'une m-séquence mère, **caractérisé en ce que** lesdits moyens de traitement (4) sont configurés pour actualiser le calcul de la réponse de chaque cellule à la fin de chaque temps de symbole (Ts), dans lequel ladite actualisation est effectuée après un certain nombre (k) de temps de symbole (Ts) pour obtenir la réponse d'une cellule q-th associée à une m-séquence translatée à l'aide d'un nombre (q) de symboles à partir de la m-séquence mère, par une corrélation croisée des données extraites de la réponse globale avec une version de ladite m-séquence translatée cycliquement retardée par ledit nombre (k) pour obtenir la réponse actuelle de ladite cellule q-th due à un nombre (N) du stimulus les plus récents, permettant de suivre l'évolution de la réponse calculée de chaque cellule en temps réel sans devoir attendre la fin d'une m-séquence.

5. Le système selon la revendication 4, dans lequel lesdits moyens de traitement (4) sont configurés pour interpréter les formes d'ondes calculées associées aux diverses cellules directement ou autrement associées à un motif composé de stimulus chromatiques lumineux, isoluminants ou d'échelles de gris qui reflète la matrice des stimulus.

6. Le système selon l'une des revendications 4 et 5, dans lequel lesdits moyens de traitement (4) comportent des moyens de stockage d'une base de données normative comprenant des résultats obtenus sur des sujets normaux, et dans lequel les moyens de traitement sont configurés pour comparer le résultat obtenu à partir du patient examiné avec ladite base de données, dans le but de fournir une indication de la déviation du sujet par rapport à la moyenne des sujets normaux en fonction de l'âge.

**7.** Le système selon l'une quelconque des revendications 4 à 6, dans lequel, entre ledit patient et lesdits moyens d'affichage (5), sont agencés des moyens séparateurs de faisceaux optiques (8), appropriés pour permettre au dit patient d'observer ladite stimulation réfléchie par lesdits moyens diviseurs plutôt que directement à partir des dits moyens d'affichage, de façon à permettre à un opérateur médical, à des fins de contrôle, d'observer la base rétinienne du patient au cours de l'examen lui-même.

**8.** Le système selon la revendication 7, comprenant en outre des moyens ophtalmoscopiques pour observer la base rétinienne du patient au cours de l'examen, lesdits moyens séparateurs de faisceaux optiques (8) et lesdits moyens d'affichage (5) étant stablement intégrés dans un seul ensemble opticomécanique avec lesdits moyens ophtalmoscopiques.

**9.** Un programme informatique pour commander un ordinateur dans le but d'exécuter le procédé selon l'une quelconque des revendications 1 à 3, quand il est mis en oeuvre dans un système selon la revendication 4.

**10.** Le programme informatique selon la revendication 9, **caractérisé en ce qu'**il est stocké sur un support mémoire.

**11.** Le programme informatique selon la revendication 9, **caractérisé en ce qu'**il est chargé dans les moyens de traitement du système selon l'une quelconque des revendications 5 à 8.

**Fig. 1a**

**Fig. 1b**

**Fig. 2**

**Fig. 3**

**Fig. 4a**

**Fig. 4b**

**Fig. 4c**

**Fig. 4d**

**Fig. 4e**

**Fig. 4f**

**Fig. 4g**

**Fig. 4h**

**Fig. 5**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4846567 A, Sutter

- US 6022107 A, Ernst Kutschbach

**Non-patent literature cited in the description**

- **E. B. BROWN et al.** Contrast luminance as parameter defining... *Etc. Ophthalmic and physiological optic,* 01 January 1996, vol. 16 n, 42-48
- **E.E. SUTTER et al.** The Field Topography of ERG Components in Man. *VISION RESEARCH,* 1992, vol. 32 (3), 433-446

- *Guideline for basic Multifocal Electroretinography (mfERG) Documenta Ophthalmologica n,* 2003, vol. 106, 105-115
- **M.A. BEARSE et al.** Imaging localized retinal dysfunction with the multifocal electroretinogram. *J. OPT. SOC. AM,* March 1996, vol. 13 (3